# EUROPEAN PATENT APPLICATION

(11) **EP 1 647 268 A1**
(43) Date of publication of application: **19.04.2006**
(21) Application number: 05291982.6
(22) Date of filing: 23.09.2005
(51) Int. Cl.: A61K 8/81, A61Q 1/06, A61Q 1/10

(54) **Cosmetic composition comprising an acrylic polymer**

(30) Priority: 15.10.2004 US 964822
(71) Applicant: L'OREAL, 75008 Paris (FR)
(72) Inventor: Yu, Wei, Edison , NJ 08820 (US); Jacquier, Isabelle, 94550 Chevilly Larue (FR)
(74) Representative: Boulard, Denis

(57) **Abstract**

The present invention relates to a cosmetic composition comprising, in a cosmetically acceptable medium:
- a polymer resulting from the copolymerization:
   a) at least one monomer A chosen from esters resulting from the reaction of (meth)acrylic acid with a monoalcohol containing from 2 to 20 carbon atoms,
   b) at least one monomer B chosen from esters derived from the reaction of methacrylic acid with a monoalcohol containing from 1 to 10 carbon atoms, and
   c) at least one monomer C chosen from N-vinyllactams and derivatives thereof, and
- an organic solvent phase comprising at least one first organic solvent, the said phase being free of lower monoalcohol containing from 1 to 5 carbon atoms or of C₃-C₄ ketone, or containing them in a weight content, relative to the total weight of the composition, of less than or equal to 15%.

## Description

The present invention relates to a cosmetic composition for making up or caring for the skin, including the scalp, of either the human face or the human body, human lips or integuments, the nails or keratin fibres, for instance the hair, the eyelashes or the eyebrows, comprising a particular acrylic polymer.

The composition may be a free or compacted powder, a foundation, a makeup rouge, an eyeshadow, a concealer product, a blusher, a lipstick, a lip balm, a lip gloss, a lip pencil, an eye pencil, an eye makeup product such as a mascara or an eyeliner, a nail varnish or a body makeup or skin colouring product.

Makeup compositions for keratin fibres, also known as eyelash "mascaras", generally consist of a wax or a mixture of waxes dispersed using at least one surfactant in an aqueous phase also containing polymers and pigments.

It is generally by means of the qualitative and quantitative choice of the waxes and polymers that the desired application specificities for the makeup compositions are adjusted, for instance their fluidity, their covering power and/or their curling power. Thus, it is possible to produce various compositions which, when applied especially to the eyelashes, induce varied effects of the type such as lengthening, curling and/or thickening.

However, with these compositions, the makeup properties, for instance the coating, the lengthening or the curling of eyelashes, are obtained when a large amount of product is applied to the eyelashes using an applicator, such as a mascara brush.

For example, document FR 83099997 discloses mascara compositions with lengthening properties, comprising a combination of an anionic film-forming polymer and of a cationic film-forming polymer; however, these compositions do not have sufficient adhesion (or attachment) to the eyelashes.

When the composition does not adhere well to the eyelashes, the user must perform several applications on the eyelashes with the brush impregnated with product, which demands that a certain amount of time be devoted in order to apply the makeup and to obtain the desired makeup results.

It is thus sought to obtain a product that has good attachment to keratin materials during application, especially good deposition of material at the end of the eyelashes so as to obtain a lengthening effect, and that allows a smooth and uniform deposition on keratin materials.

In the case of products for the lips, it is desired to obtain compositions that are capable of forming a film with good staying power, which has migration-resistance and/or transfer-resistance properties. The term "migration" means an overflowing of the composition applied to the skin or the lips beyond its initial application line.

The aim of the present invention is thus to have available a makeup composition for keratin materials, which is easy to apply to keratin materials and which rapidly produces a makeup film that has lengthening and/or staying power properties.

The inventors have found, surprisingly, that the use of a particular acrylic polymer in an organic solvent phase makes it possible to improve the adhesion properties of the composition to keratin materials. The composition is easy to apply to keratin materials and allows the deposition of a film of makeup that shows good attachment to the keratin materials, and, especially in the case of keratin fibres, a lengthening effect.

The composition of the invention may in particular be a makeup product for the human body, lips or integuments, in particular having non-therapeutic care and/or treatment properties. It is especially a lipstick or lip gloss, a makeup rouge, an eyeshadow, a tattoo product, a lip product, a makeup or care product for keratin fibres, especially the eyelashes (mascara), an eyeliner, a nail varnish, a product for artificially tanning the skin, a hair colouring or haircare product, or an eyelash care product.

More specifically, a first subject of the invention is a cosmetic composition comprising, in a cosmetically acceptable medium:
- a polymer resulting from the copolymerization:
   a) of at least one monomer A chosen from esters derived from the reaction of (meth)acrylic acid with a monoalcohol containing from 2 to 20 carbon atoms,
   b) of at least one monomer B chosen from esters derived from the reaction of methacrylic acid with a monoalcohol containing from 1 to 10 carbon atoms, and
   c) of at least one monomer C chosen from N-vinyllactams and derivatives thereof, and
- an organic solvent phase comprising at least one first organic solvent, the said phase being free of lower monoalcohol containing from 1 to 5 carbon atoms or of C₃-C₄ ketone, or containing them in a weight content, relative to the total weight of the composition, of less than or equal to 15%.

The polymer described above is also referred to as an "acrylic polymer" in the rest of the present description.

When the composition comprises a lower monoalcohol containing from 1 to 5 carbon atoms or a C₃-C₄ ketone, it preferably contains them in a content of less than or equal to 10% and preferably less than or equal to 5% by weight relative to the total weight of the composition.

The invention also relates to a process for making up the skin and/or the lips and/or the integuments, which consists in applying to the skin and/or the lips and/or the integuments the composition as defined above.

The composition according to the invention may be applied to the skin of either the face or the scalp and of the body, mucous membranes, for instance the lips, the inside of the lower eyelids, and the integuments, for instance the nails, the eyelashes, the hair, the eyebrows, or even other body hairs.

The composition according to the invention is preferably a leave-in composition.

The invention also relates to a cosmetic process for making up or caring for keratin materials, comprising the application to the said keratin materials of a composition as defined above.

Finally, a subject of the invention is the use of a polymer resulting from the copolymerization:
a) of at least one monomer A chosen from esters derived from the reaction of (meth)acrylic acid with a monoalcohol containing from 2 to 20 carbon atoms,
b) of at least one monomer B chosen from esters derived from the reaction of methacrylic acid with a monoalcohol containing from 1 to 10 carbon atoms, and
c) of at least one monomer C chosen from N-vinyllactams
to prepare a cosmetic composition capable of forming on keratin materials a film that has lengthening and/or good staying power and/or migration-resistance and/or transfer-resistance properties.

### Acrylic polymer

The acrylic polymer of the composition according to the invention is a copolymer resulting from the copolymerization:
a) of at least one monomer A chosen from esters derived from the reaction of (meth)acrylic acid with a monoalcohol containing from 2 to 20 carbon atoms,
b) of at least one monomer B chosen from esters derived from the reaction of methacrylic acid with a monoalcohol containing from 1 to 10 carbon atoms, and
c) of at least one monomer C chosen from N-vinyllactams.

The acrylic polymer of the composition according to the invention is advantageously a film-forming polymer.

The term "film-forming polymer" means a polymer capable of forming, by itself, or in the presence of an auxiliary film-forming agent, a macroscopically continuous film that adheres to keratin materials.

### Monomer A

The monomer A is chosen from esters derived from the reaction of (meth)acrylic acid with a monoalcohol containing from 2 to 20 carbon atoms.

According to one embodiment, the copolymer comprises at least one monomer A resulting from the reaction of methacrylic acid with a monoalcohol containing from 5 to 20 carbon atoms, preferably from 7 to 18 carbon atoms and better still from 10 to 18 carbon atoms.

In particular, the monoalcohol may be chosen from: 3-heptanol, 1-octanol, 2-octanol, isooctyl alcohol, 2-ethyl-1-hexanol, 1-decanol, 1-dodecanol, 1-tridecanol, 1-tetradecanol and 1-octadecanol, and mixtures thereof.

The polymer according to the invention may also comprise at least one monomer A resulting from the reaction of acrylic acid with a monoalcohol containing from 2 to 15 carbon atoms and preferably from 4 to 14 carbon atoms.

In particular, the C₂-C₁₅ monoalcohol may be chosen from: ethanol, 1-butanol, 2-butanol, 1-pentanol, 2-pentanol, 3-pentanol, 2-methyl-1-butanol, 1-hexanol, 2-hexanol, 2-methyl-1-pentanol, 3-methyl-1-pentanol, 2-ethyl-1-butanol, 3,5,5-trimethyl-1-hexanol, 3-heptanol, 1-octanol, 2-octanol, isooctyl alcohol, 2-ethyl-1-hexanol, 1-decanol, 1-dodecanol, 1-tridecanol and 1-tetradecanol, and mixtures thereof.

Advantageously, the monomer A is chosen from n-butyl acrylate, isooctyl acrylate and lauryl methacrylate (derived from the reaction of methacrylic acid and 1-dodecanol), and mixtures thereof.

Advantageously, the monomer A is present in a numerical proportion ranging from 15% to 80% and better still from 40% to 60% relative to the total number of monomers in the polymer.

### Monomer B

The monomer B is chosen from esters derived from the reaction of methacrylic acid with a monoalcohol containing from 1 to 10 carbon atoms, preferably from 1 to 6 carbon atoms and better still from 1 to 4 carbon atoms.

In particular, the monoalcohol may be chosen from methanol, ethanol, 1-propanol, 2-propanol, 1-butanol, 2-butanol, 1-pentanol, 2-pentanol and 3-pentanol, and mixtures thereof.

Preferably, the monomer B is chosen from methyl methacrylate and n-butyl methacrylate, and mixtures thereof.

Advantageously, the monomer B is present in a numerical proportion ranging from 20% to 70% and better still from 25% to 50% relative to the total number of monomers in the polymer.

### Monomer C

The monomer C is advantageously chosen from N-vinyllactams (N-substituted lactam derivatives) such as those described in document US 3 907 720, and in particular from the N-vinyllactams having the following formula: 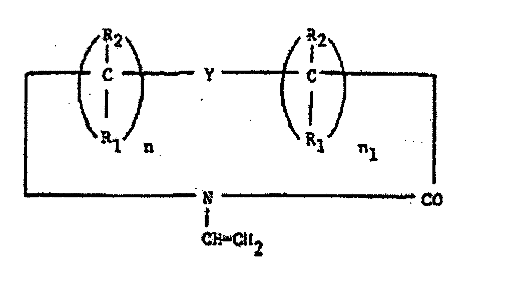 in which:
R1 and R2 independently represent a hydrogen atom, a C₁₋C₅ alkyl group such as methyl, ethyl or propyl, or an aryl group,
Y is chosen from O, S, 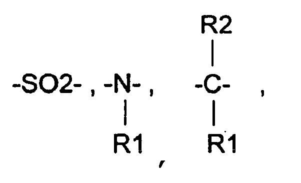 and n and n1 range from 0 to 5, with the proviso that n and n1 are not simultaneously equal to 0. Preferably, R1 and R2 independently represent a hydrogen atom or a C₁-C₅ alkyl group such as methyl, ethyl or propyl, and
Y represents

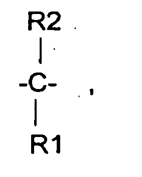

As N-vinyllactams that may be used as monomers C, mention may be made of N-vinylpyrrolidone and the N-vinyl-substituted derivatives of the following lactams: 3,3-dimethyl-1-pyrrolidone, 4,4-dimethyl-2-pyrrolidone, 3,4-dimethyl-2-pyrrolidone, 3-ethyl-2-pyrrolidone and 3,5-dimethyl-2-pyrrolidone. The monomer C is preferably N-vinylpyrrolidone.

Advantageously, the monomer C is present in a numerical proportion ranging from 1% to 15% and better still from 5% to 15% relative to the total number of monomers in the polymer.

Advantageously, the polymer is in solution or in dispersion in an organic solvent, which is preferably the "first organic solvent" of the organic solvent phase of the composition according to the invention.

The copolymer in the composition according to the invention may be prepared via conventional free-radical polymerization methods, in particular in a solvent in which the monomers are soluble.

Such copolymers and antimicrobial compositions containing them are especially described in document US 4 584 192.

The acrylic polymer in the composition according to the invention may represent from 0.01% to 30% by weight of solids (or active material), preferably from 0.05% to 20% by weight and better still from 0.05% to 15% by weight, relative to the total weight of the composition.

### Organic solvent phase

The organic solvent phase of the composition according to the invention comprises at least one first organic solvent or oil, which may especially form a fatty phase, and in particular a continuous fatty phase. The composition may be an anhydrous composition. The organic solvent phase of the composition according to the invention may comprise, besides the first organic solvent, at least one lower monoalcohol containing from 1 to 5 carbon atoms, such as ethanol or isopropanol, and/or at least one C₃-C₄ ketone such as acetone, in a weight content, relative to the total weight of the composition, of less than or equal to 15%, preferably less than or equal to 10% and more preferably less than or equal to 5% by weight relative to the total weight of the composition.

According to one embodiment, the organic solvent phase of the composition according to the invention is free of lower monoalcohols containing from 1 to 5 carbon atoms, such as ethanol or isopropanol, and of C₃-C₄ ketones such as acetone.

The term "'free' of lower monoalcohols containing from 1 to 5 carbon atoms and of ketones" means that the composition comprises less than 2%, preferably less than 1% and better still less than 0.5% by weight of the said solvents, relative to its total weight.

The first organic solvent may be volatile or non-volatile.

For the purposes of the invention, the term "volatile organic oil or solvent" means any non-aqueous medium that is capable of evaporating on contact with keratin materials in less than one hour, at room temperature and atmospheric pressure. The volatile organic solvent(s) and volatile oils of the invention are volatile organic solvents and cosmetic oils that are liquid at room temperature, with a non-zero vapour pressure at room temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg), and more particularly ranging from 1.3 Pa to 1 300 Pa (0.01 to 10 mmHg). The term "non-volatile oil" means an oil that remains on the keratin materials at room temperature and atmospheric pressure for at least several hours and that especially has a vapour pressure of less than 10⁻³ mmHg (0.13 Pa).

These oils may be hydrocarbon-based oils or silicone oils, or mixtures thereof.

The term "hydrocarbon-based oil" means an oil mainly containing hydrogen and carbon atoms and possibly oxygen, nitrogen, sulfur or phosphorus atoms. The volatile hydrocarbon-based oils may be chosen from hydrocarbon-based oils containing from 8 to 16 carbon atoms, and especially branched C₈-C₁₆ alkanes, for instance C₈-C₁₆ isoalkanes of petroleum origin (also known as isoparaffins), for instance isododecane (also known as 2,2,4,4,6-pentamethylheptane), isodecane and isohexadecane, for example the oils sold under the trade names Isopar or Permetyl, branched C₈-C₁₆ esters and isohexyl neopentanoate, and mixtures thereof. Other volatile hydrocarbon-based oils, for instance petroleum distillates, especially those sold under the name Shell Solt by the company Shell, may also be used. The volatile solvent is preferably chosen from volatile hydrocarbon-based oils containing from 8 to 16 carbon atoms, and mixtures thereof.

Volatile oils that may also be used include volatile silicones, for instance volatile linear or cyclic silicone oils, especially those with a viscosity ≤6 centistokes (6 × 10⁻⁶ m²/s) and especially containing from 2 to 10 silicon atoms, these silicones optionally comprising alkyl or alkoxy groups containing from 1 to 22 carbon atoms. As volatile silicone oils that may be used in the invention, mention may be made especially of octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane and dodecamethyl pentasiloxane, and mixtures thereof.

The volatile oil may be present in the composition according to the invention in a content ranging from 0.05% to 98% by weight and preferably from 1% to 65% by weight relative to the total weight of the composition.

The composition may also comprise at least one non-volatile oil chosen especially from non-volatile hydrocarbon-based oils and/or silicone oils.

Non-volatile hydrocarbon-based oils that may especially be mentioned include:
- hydrocarbon-based oils of plant origin, such as triglycerides consisting of fatty acid esters of glycerol, the fatty acids of which may have varied chain lengths from C₄ to C₂₄, these chains possibly being linear or branched, and saturated or unsaturated; these oils are especially wheatgerm oil, sunflower oil, grapeseed oil, sesame seed oil, maize oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, sesame seed oil, marrow oil, rapeseed oil, blackcurrant oil, evening primrose oil, millet oil, barley oil, quinoa oil, rye oil, safflower oil, candlenut oil, passion flower oil and musk rose oil; or alternatively caprylic/capric acid triglycerides such as those sold by Stearineries Dubois or those sold under the names Miglyol 810, 812 and 818 by Dynamit Nobel,
- synthetic ethers containing from 10 to 40 carbon atoms;
- linear or branched hydrocarbons of mineral or synthetic origin, such as petroleum jelly, polydecenes, hydrogenated polyisobutene such as parleam, and squalane, and mixtures thereof;
- synthetic esters such as oils of formula R₁COOR₂ in which R₁ represents a linear or branched fatty acid residue containing from 1 to 40 carbon atoms and R₂ represents an in particular branched hydrocarbon-based chain containing from 1 to 40 carbon atoms, on condition that R₅ + R₆ ≥ 10, such as, for example, purcellin oil (cetostearyl octanoate), isopropyl myristate, isopropyl palmitate, C₁₂-C₁₅ alkyl benzoate, hexyl laurate, diisopropyl adipate, isononyl isononanoate, 2-ethylhexyl palmitate, isostearyl isostearate, alkyl or polyalkyl octanoates, decanoates or ricinoleates such as propylene glycol dioctanoate; hydroxylated esters such as isostearyl lactate and diisostearyl malate; and pentaerythritol esters;
- fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;
- higher fatty acids such as oleic acid, linoleic acid or linolenic acid; and mixtures thereof.

The non-volatile silicone oils that may be used in the composition according to the invention may be non-volatile polydimethylsiloxanes (PDMSs), polydimethylsiloxanes comprising alkyl or alkoxy groups, that are pendent and/or at the end of a silicone chain, the groups each containing from 2 to 24 carbon atoms, phenylsilicones, for instance phenyltrimethicones, phenyldimethicones, phenyltri-methylsiloxydiphenylsiloxanes, diphenyldimethicones, diphenylmethyldiphenyltrisiloxanes and 2-phenylethyl-trimethylsiloxysilicates.

Preferably, the organic solvent phase comprises at least one phenylsilicone oil, which corresponds in particular to formula (A) below: 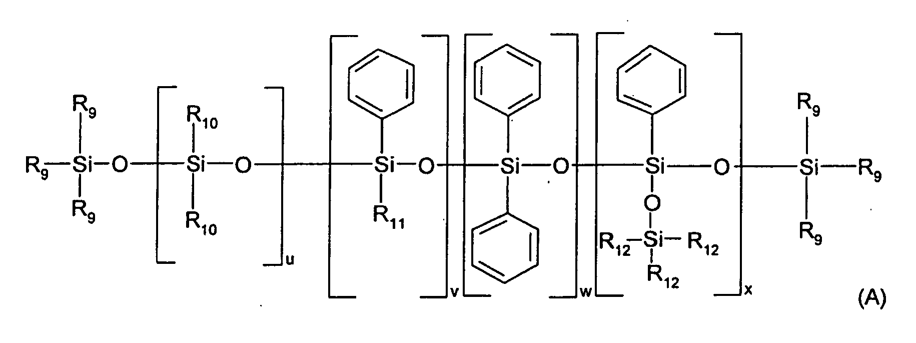 in which
- R₉ and R₁₂ are each independently a C₁-C₃₀ alkyl radical, an aryl radical or an aralkyl radical,
- R₁₀ and R₁₁ are each independently a C₁-C₃₀ alkyl radical or an aralkyl radical,
- u, v, w and x are each independently integers ranging from 0 to 900,
   with the proviso that the sum v+w+x is other than 0, and that the sum u+v+w+x ranges from 1 to 900, and in particular u+v+w+x ranges from 1 to 800.

Advantageously, R₉ is a C₁-C₂₀ alkyl radical, a phenyl radical or an aralkyl radical of the type R' -C₆H₅, R' being a C₁-C₅ alkyl, R₁₀ and R₁₁ are each independently a C₁-C₂₀ alkyl radical or an aralkyl radical of the type R' -C₆H₅, R' being a C₁-C₅ alkyl, and R₁₂ is a C₁-C₂₀ alkyl radical.

Preferably, R₉ is a methyl, ethyl, propyl, isopropyl, decyl, dodecyl or octadecyl radical, or alternatively a phenyl, tolyl, benzyl or phenethyl radical, R₁₀ and R₁₁ are each independently a methyl, ethyl, propyl, isopropyl, decyl, dodecyl or octadecyl radical or alternatively a tolyl, benzyl or phenethyl radical, and R₁₂ is a methyl, ethyl, propyl, isopropyl, decyl, dodecyl or octadecyl radical.

As phenylsilicone oils that may be used in the composition according to the invention, mention may be made of the products sold by Dow Corning under the reference DC 556, by the company Wacker under the reference Belsil PDM 1000 or by the company Shin-Etsu under the reference KF 56 or KF 58.

The organic solvent phase may represent from 0.05% to 98% by weight, preferably from 0.1% to 70% and better still from 0.5% to 60% by weight relative to the total weight of the composition.

The composition according to the invention may comprise an aqueous phase comprising water or a mixture of water and of hydrophilic organic solvent(s), for instance polyols such as glycerol, diglycerol, propylene glycol, sorbitol, pentylene glycol or polyethylene glycols, or alternatively hydrophilic C₂ ethers and C₂-C₄ aldehydes.

The aqueous phase (water or mixture of water and of hydrophilic organic solvents) may be present in the composition according to the invention in a content ranging from 1% to 95% by weight and preferably ranging from 5% to 80% by weight relative to the total weight of the composition.

When the composition according to the invention comprises such an aqueous phase, it may comprise a hydrophilic or water-soluble gelling and/or film-forming polymer.

Hydrophilic or water-soluble gelling and/or film-forming polymers that may be mentioned include:
- homopolymers or copolymers of acrylic or methacrylic acid or the salts and esters thereof, and in particular the products sold under the names "Versicol F" or "Versicol K" by the company Allied Colloid, "Ultrahold 8" by the company Ciba-Geigy, and the polyacrylic acids of Synthalen K type;
- copolymers of acrylic acid and of acrylamide sold in the form of the sodium salt thereof under the names "Reten" by the company Hercules, sodium polymethacrylate sold under the name "Darvan 7" by the company Vanderbilt, and the sodium salts of polyhydroxycarboxylic acids sold under the name "Hydagen F" by the company Henkel;
- polyacrylic acid/alkyl acrylate copolymers of the Pemulen type;
- AMPS (polyacrylamidomethylpropanesulfonic acid partially neutralized with ammonia and highly crosslinked) sold by the company Clariant;
- AMPS/acrylamide copolymers of the Sepigel or Simulgel type, sold by the company SEPPIC, and
- AMPS/polyoxyethylenated alkyl methacrylate copolymers (crosslinked or non-crosslinked), and mixtures thereof.

As other examples of water-soluble gelling and or film-forming polymers, mention may be made of:
- proteins, for instance proteins of plant origin such as wheat or soybean proteins; proteins of animal origin such as keratins, for example keratin hydrolysates and sulfonic keratins;
- anionic, cationic, amphoteric or nonionic chitin or chitosan polymers;
- cellulose polymers such as hydroxyethylcellulose, hydroxypropylcellulose, methylcellulose, ethylhydroxyethylcellulose and carboxymethylcellulose, and also quaternized cellulose derivatives;
- vinyl polymers, for instance polyvinylpyrrolidones, copolymers of methyl vinyl ether and of maleic anhydride, the copolymer of vinyl acetate and of crotonic acid, copolymers of vinylpyrrolidone and of vinyl acetate; copolymers of vinylpyrrolidone and of caprolactam; polyvinyl alcohol;
- associative polyurethanes such as the C₁₆-OE₁₂₀-C₁₆ polymer from the company Servo Delden (sold under the name Ser Ad FX1100, which is a molecule containing urethane functions and having a weight-average molecular weight of 1300), OE being an oxyethylene unit, Rheolate 205 containing urea functions, sold by the company Rheox, or Rheolate 208 or 204 (these polymers being sold in pure form) or DW 1206B from Röhm & Haas, containing a C₂₀ alkyl chain and a urethane bond, sold at an active material concentration of 20% in water. It is also possible to use solutions or dispersions of these associative polyurethanes, especially in water or in aqueous-alcoholic medium. Examples of such polymers that may be mentioned include Ser Ad FX1010, Ser Ad FX1035 and Ser Ad 1070 from the company Servo Delden, and Rheolate 255, Rheolate 278 and Rheolate 244 sold by the company Rheox. It is also possible to use the product DW 1206F and DW 1206J, and also Acrysol RM 184 or Acrysol 44 from the company Röhm & Haas, or Borchigel LW 44 from the company Borchers;
- optionally modified polymers of natural origin, such as:
   - gum arabics, guar gum, xanthan derivatives or karaya gum;
   - alginates and carrageenans;
   - glycoaminoglycans, or hyaluronic acid and its derivatives;
   - shellac resin, sandarac gum, dammar resins, elemi gums and copal resins;
   - deoxyribonucleic acid;
   - mucopolysaccharides such as hyaluronic acid and chondroitin sulfate, and mixtures thereof.

The hydrophilic gelling agents may be present in the composition according to the invention in a content ranging from 0.05% to 40% by weight, preferably from 0.5% to 20% and better still from 1% to 15% by weight relative to the total weight of the composition.

The composition according to the invention may contain emulsifying surfactants, present especially in a proportion ranging from 0.5% to 30% by weight, better still from 2% to 20% and even better still from 3% to 15% by weight relative to the total weight of the composition. These surfactants may be chosen from anionic, cationic and nonionic surfactants. Reference may be made to the document "Encyclopedia of Chemical Technology, Kirk-Othmer", Volume 22, pp. 333-432, 3rd Edition, 1979, Wiley, for the definition of the properties and functions (emulsifying) of surfactants, in particular pp. 347-377 of this reference, for the anionic and nonionic surfactants.

The surfactants preferably used in the composition according to the invention are chosen from:
a) Nonionic surfactants with an HLB of greater than or equal to 8 at 25°C, used alone or as a mixture; mention may be made especially of:
   - oxyethylenated and/or oxypropylenated ethers (which may comprise from 1 to 150 oxyethylene and/or oxypropylene groups) of glycerol;
   - oxyethylenated and/or oxypropylenated ethers (which may comprise from 1 to 150 oxyethylene and/or oxypropylene groups) of fatty alcohols (especially of C8-C24 and preferably C12-C18 alcohol), such as oxyethylenated cetearyl alcohol ether containing 30 oxyethylene groups (CTFA name "Ceteareth-30") and the oxyethylenated ether of the mixture of C12-C15 fatty alcohols comprising 7 oxyethylene groups (CTFA name "C12-15 Pareth-7" sold under the name "Neodol 25-7® " by Shell Chemicals);
   - fatty acid esters (especially of a C8-C24 and preferably C16-C22 acid) of polyethylene glycol (which may comprise from 1 to 150 ethylene glycol units), such as PEG-50 stearate and PEG-40 monostearate sold under the name Myrj 52P by the company ICI Uniqema;
   - fatty acid esters (especially of a C8-C24 and preferably C16-C22 acid) of oxyethylenated and/or oxypropylenated glyceryl ethers (which may comprise from 1 to 150 oxyethylene and/or oxypropylene groups), for instance PEG-200 glyceryl monostearate sold under the name "Simulsol 220 TM" by the company SEPPIC; glyceryl stearate polyethoxylated with 30 ethylene oxide groups, for instance the product Tagat S sold by the company Goldschmidt, glyceryl oleate polyethoxylated with 30 ethylene oxide groups, for instance the product Tagat O sold by the company Goldschmidt, glyceryl cocoate polyethoxylated with 30 ethylene oxide groups, for instance the product Varionic LI 13 sold by the company Sherex, glyceryl isostearate polyethoxylated with 30 ethylene oxide groups, for instance the product Tagat L sold by the company Goldschmidt, and glyceryl laurate polyethoxylated with 30 ethylene oxide groups, for instance the product Tagat I from the company Goldschmidt;
   - fatty acid esters (especially of a C8-C24 and preferably C16-C22 acid) of oxyethylenated and/or oxypropylenated sorbitol ethers (which may comprise from 1 to 150 oxyethylene and/or oxypropylene groups), for instance polysorbate 60 sold under the name "Tween 60" by the company Uniqema;
   - dimethicone copolyol, such as the product sold under the name "Q2-5220" by the company Dow Corning,
   - dimethicone copolyol benzoate (Finsolv SLB 101 and 201 by the company Finetex),
   - copolymers of propylene oxide and of ethylene oxide, also known as EO/PO polycondensates, for instance the polyethylene glycol/polypropylene glycol/polyethylene glycol triblock polycondensates sold under the name "Synperonic", for instance "Synperonic PE/L44" and "Synperonic PE/F127", by the company ICI, and mixtures thereof;
   - and mixtures thereof.
b) Nonionic surfactants with an HLB value of less than 8 at 25°C, optionally combined with one or more nonionic surfactants with an HLB value of greater than 8 at 25°C, as mentioned above, such as:
   - saccharide esters and ethers, such as sucrose stearate, sucrose cocoate and sorbitan stearate, and mixtures thereof, for instance Arlatone 2121 sold by the company ICI;
   - fatty acid esters (especially of a C8-C24 and preferably C16-C22 acid) of polyols, especially of glycerol or of sorbitol, such as glyceryl stearate, glyceryl stearate such as the product sold under the name Tegin M by the company Goldschmidt, glyceryl laurate such as the product sold under the name Imwitor 312 by the company Hüls, polyglyceryl-2 stearate, sorbitan tristearate or glyceryl ricinoleate;
   - the mixture of cyclomethicone/dimethicone copolyol sold under the name "Q2-3225C" by the company Dow Corning.
c) Anionic surfactants such as:
   - C₁₆-C₃₀ fatty acid salts, especially those derived from amines, for instance triethanolamine stearate and/or amino-2 methyl-2 propane diol-1,3;
   - polyoxyethylenated fatty acid salts, especially those derived from amines or alkali metal salts, and mixtures thereof;
   - phosphoric esters and salts thereof, such as "DEA oleth-10 phosphate" (Crodafos N 10N from the company Croda);
   - sulfosuccinates such as "Disodium PEG-5 citrate lauryl sulfosuccinate" and "Disodium ricinoleamido MEA sulfosuccinate";
   - alkyl ether sulfates, such as sodium lauryl ether sulfate;
   - isethionates;
   - acylglutamates such as "Disodium hydrogenated tallow glutamate" (Amisoft HS-21 R sold by the company Ajinomoto), and mixtures thereof.

Triethanolamine stearate is most particularly suitable for the invention. This surfactant is generally obtained by simple mixing of stearic acid and triethanolamine.

Surfactants that allow an oil-in-water or wax-in-water emulsion to be obtained are preferably used.

The composition may comprise at least one additional film-forming polymer other than the acrylic polymer described above.

Thus, according to one embodiment, a subject of the invention is a cosmetic composition comprising, in a cosmetically acceptable medium:
- a polymer resulting from the copolymerization:
   a) of at least one monomer A chosen from esters derived from the reaction of (meth)acrylic acid with a monoalcohol containing from 2 to 20 carbon atoms,
   b) of at least one monomer B chosen from esters derived from the reaction of methacrylic acid with a monoalcohol containing from 1 to 10 carbon atoms, and
   c) of at least one monomer C chosen from
      N-vinyllactams and derivatives thereof,
- at least one additional film-forming polymer.

Among the additional film-forming polymers that may be used in the composition of the present invention, mention may be made of synthetic polymers, of radical-mediated type or of polycondensate type, and polymers of natural origin, and mixtures thereof.

The expression "radical-mediated film-forming polymer" means a polymer obtained by polymerization of monomers containing unsaturation, in particular ethylenic unsaturation, each monomer being capable of homopolymerizing (unlike polycondensates).

The film-forming polymers of radical-mediated type may be, in particular, vinyl polymers or copolymers, in particular acrylic polymers.

The vinyl film-forming polymers can result from the polymerization of monomers containing ethylenic unsaturation and containing at least one acidic group and/or esters of these acidic monomers and/or amides of these acidic monomers.

Monomers bearing an acidic group which may be used are α,β-ethylenic unsaturated carboxylic acids such as acrylic acid, methacrylic acid, crotonic acid, maleic acid or itaconic acid. (Meth)acrylic acid and crotonic acid are preferably used, and more preferably (meth)acrylic acid.

The esters of acidic monomers are advantageously chosen from (meth)acrylic acid esters (also known as (meth)acrylates), especially (meth)acrylates of an alkyl, in particular of a C₁-C₃₀ and preferably C₁-C₂₀ alkyl, (meth)acrylates of an aryl, in particular of a C₆-C₁₀ aryl, and (meth)acrylates of a hydroxyalkyl, in particular of a C₂-C₆ hydroxyalkyl.

Among the alkyl (meth)acrylates that may be mentioned are methyl methacrylate, ethyl methacrylate, butyl methacrylate, isobutyl methacrylate, 2-ethylhexyl methacrylate, lauryl methacrylate and cyclohexyl methacrylate.

Among the hydroxyalkyl (meth)acrylates that may be mentioned are hydroxyethyl acrylate, 2-hydroxypropyl acrylate, hydroxyethyl methacrylate and 2-hydroxypropyl methacrylate.

Among the aryl (meth)acrylates that may be mentioned are benzyl acrylate and phenyl acrylate.

The (meth)acrylic acid esters that are particularly preferred are the alkyl (meth)acrylates.

According to the present invention, the alkyl group of the esters may be either fluorinated or perfluorinated, i.e. some or all of the hydrogen atoms of the alkyl group are substituted with fluorine atoms.

Examples of amides of the acid monomers that may be mentioned are (meth)acrylamides, and especially N-alkyl(meth)acrylamides, in particular of a C₂-C₁₂ alkyl. Among the N-alkyl(meth)acrylamides that may be mentioned are N-ethylacrylamide, N-t-butylacrylamide, N-t-octylacrylamide and N-undecylacrylamide.

The vinyl film-forming polymers may also result from the homopolymerization or copolymerization of monomers chosen from vinyl esters and styrene monomers. In particular, these monomers may be polymerized with acid monomers and/or esters thereof and/or amides thereof, such as those mentioned above.

Examples of vinyl esters that may be mentioned are vinyl acetate, vinyl neodecanoate, vinyl pivalate, vinyl benzoate and vinyl t-butylbenzoate.

Styrene monomers that may be mentioned are styrene and α-methylstyrene.

Among the film-forming polycondensates that may be mentioned are polyurethanes, polyesters, polyesteramides, polyamides, epoxyester resins and polyureas.

The polyurethanes may be chosen from anionic, cationic, nonionic and amphoteric polyurethanes, polyurethane-acrylics, polyurethane-polyvinylpyrrolidones, polyester-polyurethanes, polyether-polyurethanes, polyureas and polyurea/polyurethanes, and mixtures thereof.

The polyesters may be obtained, in a known manner, by polycondensation of dicarboxylic acids with polyols, in particular diols.

The dicarboxylic acid may be aliphatic, alicyclic or aromatic. Examples of such acids that may be mentioned are: oxalic acid, malonic acid, dimethylmalonic acid, succinic acid, glutaric acid, adipic acid, pimelic acid, 2,2-dimethylglutaric acid, azelaic acid, suberic acid, sebacic acid, fumaric acid, maleic acid, itaconic acid, phthalic acid, dodecanedioic acid, 1,3-cyclohexanedicarboxylic acid, 1,4-cyclohexanedicarboxylic acid, isophthalic acid, terephthalic acid, 2,5-norbornanedicarboxylic acid, diglycolic acid, thiodipropionic acid, 2,5-naphthalenedicarboxylic acid or 2,6-naphthalenedicarboxylic acid. These dicarboxylic acid monomers may be used alone or as a combination of at least two dicarboxylic acid monomers. Among these monomers, the ones preferentially chosen are phthalic acid, isophthalic acid and terephthalic acid.

The diol may be chosen from aliphatic, alicyclic and aromatic diols. The diol used is preferably chosen from: ethylene glycol, propylene glycol, diethylene glycol, triethylene glycol, 1,3-propanediol, cyclohexanedimethanol and 4-butanediol. Other polyols that may be used are glycerol, pentaerythritol, sorbitol and trimethylolpropane.

The polyesteramides may be obtained in a manner analogous to that of the polyesters, by polycondensation of diacids with diamines or amino alcohols. Diamines that may be used are ethylenediamine, hexamethylenediamine and meta- or para-phenylenediamine. An amino alcohol that may be used is monoethanolamine.

The polyester may also comprise at least one monomer bearing at least one group -SO₃M, with M representing a hydrogen atom, an ammonium ion NH₄⁺ or a metal ion such as, for example, an Na⁺, Li⁺, K⁺, Mg²⁺, Ca²⁺, Cu²⁺, Fe²⁺ or Fe³⁺ ion. A difunctional aromatic monomer comprising such a group -SO₃M may be used in particular.

The aromatic nucleus of the difunctional aromatic monomer also bearing a group -SO₃M as described above may be chosen, for example, from benzene, naphthalene, anthracene, biphenyl, oxybiphenyl, sulfonylbiphenyl and methylenebiphenyl nuclei. As examples of difunctional aromatic monomers also bearing a group -SO₃M, mention may be made of: sulfoisophthalic acid, sulfoterephthalic acid, sulfophthalic acid, 4-sulfonaphthalene-2,7-dicarboxylic acid.

The copolymers preferably used are those based on isophthalate/sulfoisophthalate, and more particularly copolymers obtained by condensation of diethylene glycol, cyclohexanedimethanol, isophthalic acid and sulfoisophthalic acid.

The polymers of natural origin, optionally modified, may be chosen from shellac resin, sandarac gum, dammar resins, elemi gums, copal resins and cellulose polymers, and mixtures thereof.

According to a first embodiment of the composition according to the invention, the film-forming polymer may be a water-soluble polymer and may be present in an aqueous phase of the composition; the polymer is thus solubilized in the aqueous phase of the composition. Examples of water-soluble film-forming polymers that may be mentioned are:
- proteins, for instance proteins of plant origin such as wheat proteins and soybean proteins; proteins of animal origin such as keratins, for example keratin hydrolysates and sulfonic keratins;
- polymers of cellulose such as hydroxyethylcellulose, hydroxypropylcellulose, methylcellulose, ethylhydroxyethylcellulose and carboxymethylcellulose, and quaternized cellulose derivatives;
- acrylic polymers or copolymers, such as polyacrylates or polymethacrylates;
- vinyl polymers, for instance polyvinylpyrrolidones, copolymers of methyl vinyl ether and of malic anhydride, the copolymer of vinyl acetate and of crotonic acid, copolymers of vinylpyrrolidone and of vinyl acetate; copolymers of vinylpyrrolidone and of caprolactam; polyvinyl alcohol;
- and mixtures thereof.

According to another embodiment of the composition according to the invention, the film-forming polymer may be a polymer dissolved in a liquid fatty phase comprising organic solvents or oils such as those described above (the film-forming polymer is thus said to be a liposoluble polymer). For the purposes of the invention, the expression "liquid fatty phase" means a fatty phase which is liquid at room temperature (25°C) and atmospheric pressure (760 mmHg, i.e. 10⁵ Pa), composed of one or more fatty substances that are liquid at room temperature, such as oils described above, which are generally mutually compatible.

The liquid fatty phase preferably comprises a volatile oil, optionally mixed with a non-volatile oil, the oils possibly being chosen from those mentioned above.

Examples of liposoluble polymers which may be mentioned are copolymers of vinyl ester (the vinyl group being directly linked to the oxygen atom of the ester group and the vinyl ester containing a saturated, linear or branched hydrocarbon-based radical of 1 to 19 carbon atoms, linked to the carbonyl of the ester group) and of at least one other monomer which may be a vinyl ester (other than the vinyl ester already present), an α-olefin (containing from 8 to 28 carbon atoms), an alkyl vinyl ether (in which the alkyl group comprises from 2 to 18 carbon atoms) or an allylic or methallylic ester (containing a saturated, linear or branched hydrocarbon-based radical of 1 to 19 carbon atoms, linked to the carbonyl of the ester group).

These copolymers may be crosslinked with the aid of crosslinking agents, which may be either of the vinyl type or of the allylic or methallylic type, such as tetraallyloxyethane, divinylbenzene, divinyl octanedioate, divinyl dodecanedioate and divinyl octadecanedioate.

Examples of these copolymers which may be mentioned are the following copolymers: vinyl acetate/allyl stearate, vinyl acetate/vinyl laurate, vinyl acetate/vinyl stearate, vinyl acetate/octadecene, vinyl acetate/octadecyl vinyl ether, vinyl propionate/allyl laurate, vinyl propionate/vinyl laurate, vinyl stearate/1-octadecene, vinyl acetate/1-dodecene, vinyl stearate/ethyl vinyl ether, vinyl propionate/cetyl vinyl ether, vinyl stearate/allyl acetate, vinyl 2,2-dimethyloctanoate/vinyl laurate, allyl 2,2-dimethylpentanoate/vinyl laurate, vinyl dimethylpropionate/vinyl stearate, allyl dimethylpropionate/vinyl stearate, vinyl propionate/vinyl stearate, crosslinked with 0.2% divinylbenzene, vinyl dimethylpropionate/vinyl laurate, crosslinked with 0.2% divinylbenzene, vinyl acetate/octadecyl vinyl ether, crosslinked with 0.2% tetaallyloxyethane, vinyl acetate/allyl stearate, crosslinked with 0.2% divinylbenzene, vinyl acetate/1-octadecene, crosslinked with 0.2% divinylbenzene, and allyl propionate/allyl stearate, crosslinked with 0.2% divinylbenzene.

Examples of liposoluble film-forming polymers which may also be mentioned are liposoluble copolymers, and in particular those resulting from the copolymerization of vinyl esters containing from 9 to 22 carbon atoms or of alkyl acrylates or methacrylates, and alkyl radicals containing from 10 to 20 carbon atoms.

Such liposoluble copolymers may be chosen from polyvinyl stearate, polyvinyl stearate crosslinked with the aid of divinylbenzene, of diallyl ether or of diallyl phthalate copolymers, polystearyl (meth)acrylate, polyvinyl laurate and polylauryl (meth)acrylate copolymers, it being possible for these poly(meth)acrylates to be crosslinked with the aid of ethylene glycol dimethacrylate or tetraethylene glycol dimethacrylate.

The liposoluble copolymers defined above are known and are described in particular in patent application FR-A-2 232 303; they may have a weight-average molecular weight ranging from 2 000 to 500 000 and preferably from 4 000 to 200 000.

As liposoluble film-forming polymers which may be used in the invention, mention may also be made of polyalkylenes and in particular copolymers of C₂-C₂₀ alkenes, such as polybutene, alkylcelluloses with a linear or branched, saturated or unsaturated C₁-C₈ alkyl radical, for instance ethylcellulose and propylcellulose, copolymers of vinylpyrrolidone (VP) and in particular copolymers of vinylpyrrolidone and of C₂ to C₄₀ and better still C₃ to C₂₀ alkene. As examples of VP copolymers which may be used in the invention, mention may be made of the copolymers of VP/vinyl acetate, VP/ethyl methacrylate, butylated polyvinylpyrrolidone (PVP), VP/ethyl methacrylate/methacrylic acid, VP/eicosene, VP/hexadecene, VP/triacontene, VP/styrene or VP/acrylic acid/lauryl methacrylate.

The film-forming polymer may also be present in the composition in the form of particles dispersed in an aqueous phase or in a non-aqueous solvent phase, which is generally known as a latex or pseudolatex. The techniques for preparing these dispersions are well known to those skilled in the art.

Aqueous dispersions of film-forming polymers which may be used are the acrylic dispersions sold under the names Neocryl XK-90®, Neocryl A-1070®, Neocryl A-1090®, Neocryl BT-62®, Neocryl A-1079® and Neocryl A-523® by the company Avecia-Neoresins, Dow Latex 432® by the company Dow Chemical, Daitosol 5000 AD® or Daitosol 5000 SJ by the company Daito Kasey Kogyo; Syntran 5760 by the company Interpolymer or the aqueous dispersions of polyurethane sold under the names Neorez R-981® and Neorez R-974® by the company Avecia-Neoresins, Avalure UR-405®, Avalure UR-410®, Avalure UR-425®, Avalure UR-450®, Sancure 875®, Sancure 861®, Sancure 878® and Sancure 2060® by the company Goodrich, Impranil 85® by the company Bayer and Aquamere H-1511® by the company Hydromer; the sulfopolyesters sold under the brand name "Eastman AQ®" by the company Eastman Chemical Products, vinyl dispersions, for instance "Mexomer PAM" and also acrylic dispersions in isododecane, for instance "Mexomer PAP" by the company Chimex.

According to one particular embodiment, the composition according to the invention comprises, as additional film-forming polymers, a combination of a cationic polymer and an anionic polymer.

The cationic polymer may be chosen from quaternary cellulose ether derivatives, copolymers of cellulose with a water-soluble quaternary ammonia monomer, cyclopolymers, cationic polysaccharides, cationic silicone polymers, vinylpyrrolidone/dialkyl-aminoalkyl acrylate or methacrylate quaternized or non-quaternized copolymers, quaternary polymers of vinylpyrrolidone and of vinylimidazole, and polyaminoamides, and mixtures thereof.

Preferably, the cationic polymer is a hydroxy(C₁-C₄)alkylcellulose comprising quaternary ammonium groups.

The anionic polymer is advantageously chosen from:
A) homopolymers or copolymers of acrylic or methacrylic acid or salts thereof, copolymers of acrylic acid and of acrylamide and salts thereof, and the sodium salts of polyhydroxycarboxylic acids;
B) copolymers of acrylic or methacrylic acid with a monoethylenic monomer such as ethylene, styrene, vinyl esters, acrylic or methacrylic acid esters, optionally grafted onto a polyalkylene glycol such as polyethylene glycol; copolymers of this type comprising in their chain an optionally N-alkylated and/or hydroxyalkylated acrylamide unit, copolymers of acrylic acid and of a C₁₋C₄ alkyl methacrylate, and terpolymers of vinylpyrrolidone, of acrylic acid and of a C₁-C₂₀ alkyl methacrylate;
C) copolymers derived from crotonic acid, such as those comprising in their chain vinyl acetate or propionate units and optionally other monomers such as allylic or methallylic esters, a vinyl ether or vinyl ester of a linear or branched saturated carboxylic acid with a long hydrocarbon-based chain, such as those comprising at least 5 carbon atoms, these polymers possibly being grafted;
D) polymers derived from maleic, fumaric or itaconic acid or anhydride with vinyl esters, vinyl ethers, vinyl halides, phenylvinyl derivatives or acrylic acid and esters thereof; copolymers of maleic, citraconic or itaconic anhydride and of an allylic or methallylic ester optionally comprising an acrylamide or methacrylamide group, an α-olefin, acrylic or methacrylic esters, acrylic or methacrylic acid or vinylpyrrolidone in their chain, the anhydride functions being monoesterified or monoamidated;
E) polyacrylamides comprising carboxylate groups,
F) deoxyribonucleic acid;
G) copolymers of at least one dicarboxylic acid, of at least one diol and of at least one difunctional aromatic monomer bearing a group -SO₃M with M representing a hydrogen atom, an ammonium ion NH₄⁺ or a metal ion;
   - and mixtures thereof.

The anionic polymers which are more particularly preferred are chosen from non-crosslinked anionic polymers such as the methyl vinyl ether/monoesterified maleic anhydride copolymers sold under the name Gantrez ES 425 by the company ISP, the acrylic acid/ethyl acrylate/N-tert-butylacrylamide terpolymers sold under the name Ultrahold Strong by the company BASF, the copolymers of methacrylic acid and of methyl methacrylate sold under the name Eudragit L by the company Rohm Pharma, the vinyl acetate/vinyl tert-butylbenzoate/crotonic acid terpolymers and the crotonic acid/vinyl acetate/vinyl neododecanoate terpolymers sold under the name Resin 28-29-30 by the company National Starch, the copolymers of methacrylic acid and of ethyl acrylate sold under the name Luvimer MAEX or MAE by the company BASF, the vinylpyrrolidone/acrylic acid/lauryl methacrylate terpolymers sold under the name Acrylidone LM by the company ISP and the acrylic or methacrylic acid homopolymers sold, for example, under the name Versicol E 5 or poly(sodium methacrylate) sold under the name Darvan 7 by the company Vanderbilt, and mixtures thereof.

The anionic polymer is preferably a sodium polymethacrylate.

The additional film-forming polymer(s) may be present in a solids content ranging from 0.1% to 60% by weight, preferably from 0.5% to 40% by weight and better still from 1% to 30% by weight relative to the total weight of the composition.

The composition according to the invention may comprise a plasticizer that promotes the formation of a film with the film-forming polymer. Such a plasticizer may be chosen from any of the compounds known to those skilled in the art as being capable of filling the desired function.

The additional film-forming polymer may be combined with one or more auxiliary film-forming agents. Such a film-forming agent may be chosen from any of the compounds known to those skilled in the art as being capable of filling the desired function, and may be chosen especially from plasticizers and coalescers.

The composition according to the invention may also comprise at least one fatty substance that is solid at room temperature, chosen especially from waxes and pasty fatty substances, and mixtures thereof. These fatty substances may be of animal, plant, mineral or synthetic origin.

The composition according to the invention may comprise at least one wax. For the purposes of the present invention, the term "wax" means a lipophilic compound, which is solid at room temperature (25°C), which undergoes a reversible solid/liquid change of state, and which has a melting point of greater than or equal to 30°C, which may be up to 120°C.

The melting point of the wax may be measured using a differential scanning calorimeter (D.S.C.), for example the calorimeter sold under the name DSC 30 by the company Mettler.

The waxes may be hydrocarbon-based waxes, fluoro waxes and/or silicone waxes, and may be of plant, mineral, animal and/or synthetic origin. In particular, the waxes have a melting point of greater than 25°C and better still greater than 45°C.

Hydrocarbon-based waxes such as beeswax, lanolin wax and Chinese insect waxes; rice wax, carnauba wax, candelilla wax, ouricury wax, esparto grass wax, cork fibre wax, sugar cane wax, Japan wax and sumach wax; montan wax, microcrystalline waxes, paraffins and ozokerite; polyethylene waxes, the waxes obtained by Fisher-Tropsch synthesis and waxy copolymers, and also esters thereof, may especially be used.

Mention may also be made of waxes obtained by catalytic hydrogenation of animal or plant oils containing linear or branched C₈-C₃₂ fatty chains.

Among these waxes that may especially be mentioned are hydrogenated jojoba oil, isomerized jojoba oil such as the trans-isomerized partially hydrogenated jojoba oil manufactured or sold by the company Desert Whale under the commercial reference Iso-Jojoba-50® , hydrogenated sunflower oil, hydrogenated castor oil, hydrogenated coconut oil, hydrogenated lanolin oil and bis(1,1,1-trimethylolpropane) tetrastearate sold under the name "Hest 2T-4S" by the company Heterene, bis(1,1,1-trimethylolpropane) tetrabehenate sold under the name Hest 2T-4B by the company Heterene.

Mention may also be made of silicone waxes, for instance alkyl or alkoxy dimethicones containing from 16 to 45 carbon atoms, and fluoro waxes.

The waxes obtained by hydrogenation of olive oil esterified with stearyl alcohol, sold under the name "Phytowax Olive 18 L57" or else the waxes obtained by hydrogenation of castor oil esterified with cetyl alcohol sold under the names "Phytowax ricin 16L64 and 22L73" by the company Sophim may also be used. Such waxes are described in patent application FR-A-2 792 190.

According to one particular embodiment, the compositions according to the invention may comprise at least one "tacky" wax, i.e. a wax with a tack of greater than or equal to 0.7 N.s and a hardness of less than or equal to 3.5 MPa.

Using a tacky wax may especially make it possible to obtain a cosmetic composition that applies easily to keratin fibres, attaches well to the keratin fibres and leads to the formation of a smooth, uniform and thickening makeup result.

The tacky wax used may especially have a tack ranging from 0.7 N.s to 30 N.s, in particular greater than or equal to 1 N.s, especially ranging from 1 N.s to 20 N.s, in particular greater than or equal to 2 N.s, especially ranging from 2 N.s to 10 N.s and in particular ranging from 2 N.s to 5 N.s.

The tack of the wax is determined by measuring the change in force (compression force or stretching force) as a function of time, at 20°C, using the texturometer sold under the name "TA-2i® " by the company Rheo, equipped with a conical acrylic polymer spindle forming an angle of 45°.

The measuring protocol is as follows:

The wax is melted at a temperature equal to the melting point of the wax + 10°C. The molten wax is poured into a container 25 mm in diameter and 20 mm deep. The wax is recrystallized at room temperature (25°C) for 24 hours such that the surface of the wax is flat and smooth, and the wax is then stored for at least 1 hour at 20°C before measuring the tack.

The texturometer spindle is displaced at a speed of 0.5 mm/s then penetrates the wax to a penetration depth of 2 mm. When the spindle has penetrated the wax to a depth of 2 mm, the spindle is held still for 1 second (corresponding to the relaxation time) and is then withdrawn at a speed of 0.5 mm/s.

During the relaxation time, the force (compression force) decreases greatly until it becomes zero, and then, during the withdrawal of the spindle, the force (stretching force) becomes negative and then rises again to the value 0. The tack corresponds to the integral of the curve of the force as a function of time for the part of the curve corresponding to negative values of the force (stretching force). The tack value is expressed in N.s.

The tacky wax that may be used generally has a hardness of less than or equal to 3.5 MPa, in particular ranging from 0.01 MPa to 3.5 MPa, especially ranging from 0.05 MPa to 3 MPa or even ranging from 0.1 MPa to 2.5 MPa.

The hardness is measured according to the protocol described previously.

Tacky waxes that may be used include a C₂₀-C₄₀ alkyl (hydroxystearyloxy)stearate (the alkyl group containing from 20 to 40 carbon atoms), alone or as a mixture, in particular a C₂₀-C₄₀ alkyl 12-(12'-,-of formula (II): 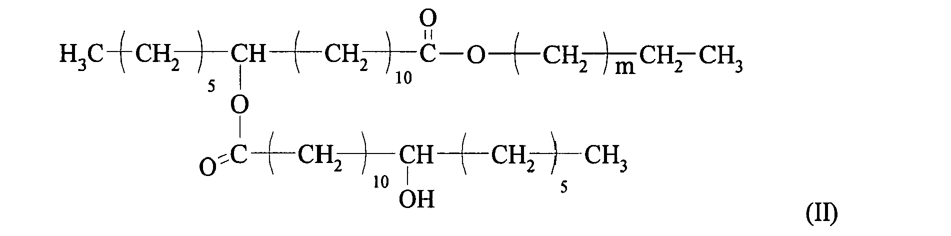 in which m is an integer ranging from 18 to 38, or a mixture of compounds of formula (II).

Such a wax is especially sold under the names "Kester Wax K 82 P® " and "Kester Wax K 80 P® " by the company Koster Keunen.

The waxes mentioned above generally have a starting melting point of less than 45°C.

It is also possible to use the microcrystalline wax sold under the reference SP18 by the company Strahl & Pitsch, which has a hardness of about 0.46 MPa and a tack value of about 1 N.s.

The wax(es) may be present in the form of an aqueous wax microdispersion. The term "aqueous wax microdispersion" means an aqueous dispersion of wax particles, in which the size of the said wax particles is less than or equal to about 1 µm.

Wax microdispersions are stable dispersions of colloidal wax particles, and are described especially in "Microemulsions Theory and Practice", L.M. Prince Ed., Academic Press (1977) pages 21-32.

In particular, these wax microdispersions may be obtained by melting the wax in the presence of a surfactant, and optionally of a portion of water, followed by gradual addition of hot water with stirring. The intermediate formation of an emulsion of the water-in-oil type is observed, followed by a phase inversion, with final production of a microemulsion of the oil-in-water type. On cooling, a stable microdispersion of solid wax colloidal particles is obtained.

The wax microdispersions may also be obtained by stirring the mixture of wax, surfactant and water using stirring means such as ultrasound, a highpressure homogenizer or turbomixers.

The particles of the wax microdispersion preferably have mean sizes of less than 1 µm (especially ranging from 0.02 µm to 0.99 µm) and preferably less than 0.5 µm (especially ranging from 0.06 µm to 0.5 µm).

These particles consist essentially of a wax or a mixture of waxes. However, they may comprise a small proportion of oily and/or pasty fatty additives, a surfactant and/or a common liposoluble additive/active agent.

The composition according to the invention may contain at least one fatty compound that is pasty at room temperature. For the purposes of the invention, the expression "pasty fatty substance" means fatty substances with a melting point ranging from 20 to 55°C, preferably 25 to 45°C, and/or a viscosity at 40°C ranging from 0.1 to 40 Pa.s (1 to 400 poises), preferably 0.5 to 25 Pa.s, measured using a Contraves TV or Rheomat 80 viscometer, equipped with a spindle rotating at 60 Hz. A person skilled in the art can select the spindle for measuring the viscosity from the spindles MS-r3 and MS-r4, on the basis of his general knowledge, so as to be able to carry out the measurement of the pasty compound tested.

These fatty substances are preferably hydrocarbon-based compounds, optionally of polymeric type; they can also be chosen from silicone compounds; they may also be in the form of a mixture of hydrocarbon-based compounds and/or silicone compounds. In the case of a mixture of different pasty fatty substances, the hydrocarbon-based pasty compounds (containing mainly hydrogen and carbon atoms and optionally ester groups) are preferably used in major proportion.

Among the pasty compounds which may be used in the composition according to the invention, mention may be made of lanolins and lanolin derivatives such as acetylated lanolins or oxypropylenated lanolins or isopropyl lanolate, having a viscosity of from 18 to 21 Pa.s, preferably 19 to 20.5 Pa.s, and/or a melting point of from 30 to 55°C, and mixtures thereof. It is also possible to use esters of fatty acids or of fatty alcohols, in particular those containing from 20 to 65 carbon atoms (melting point of about from 20 to 35°C and/or viscosity at 40°C ranging from 0.1 to 40 Pa.s), such as triisostearyl or cetyl citrate; arachidyl propionate; polyvinyl laurate; cholesterol esters, such as triglycerides of plant origin, such as hydrogenated plant oils, viscous polyesters such as poly(12-hydroxystearic acid), and mixtures thereof.

Mention may also be made of pasty silicone fatty substances such as polydimethylsiloxanes (PDMSs) containing pendent chains of the alkyl or alkoxy type containing from 8 to 24 carbon atoms, and having a melting point of 20-55°C, such as stearyldimethicones, in particular those sold by Dow Corning under the trade names DC2503 and DC25514, and mixtures thereof.

The nature and amount of the solid fatty substances depend on the desired mechanical properties and textures.

As a guide, the composition may contain from 0.1% to 50% by weight, better still from 1% to 40% and even better still from 5% to 30% by weight of waxes, relative to the total weight of the composition. The pasty fatty substance may be present in the composition according to the invention in a content ranging from 0.01% to 60% by weight, preferably ranging from 0.5% to 45% by weight and better still ranging from 2% to 30% by weight, in the composition, relative to the total weight of the composition.

The composition according to the invention may also comprise one or more dyestuffs chosen from water-soluble or liposoluble dyes, and pulverulent dyestuffs, for instance pigments, nacres and flakes that are well known to those skilled in the art. The dyestuffs may be present in the composition in a content ranging from 0.01% to 50% by weight and preferably from 0.01% to 30% by weight relative to the weight of the composition.

The term "pigments" should be understood as meaning white or coloured, mineral or organic particles of any form, which are insoluble in physiological medium, and which are intended to colour the composition.

The term "nacres" should be understood as meaning iridescent particles of any form, especially produced by certain molluscs in their shell, or alternatively synthesized.

The pigments may be white or coloured, and mineral and/or organic. Among the mineral pigments that may be mentioned are titanium dioxide, optionally surface-treated, zirconium oxide or cerium oxide, and also zinc oxide, iron oxide (black, yellow or red) or chromium oxide, manganese violet, ultramarine blue, chromium hydrate and ferric blue, and metal powders, for instance aluminium powder or copper powder. Among the organic pigments that may be mentioned are carbon black, pigments of D & C type, and lakes based on cochineal carmine or on barium, strontium, calcium or aluminium.

Mention may also be made of pigments with an effect, such as particles comprising a natural or synthetic, organic or mineral substrate, for instance glass, acrylic resins, polyester, polyurethane, polyethylene terephthalate, ceramics or aluminas, the said substrate possibly being coated with metallic substances, for instance aluminium, gold, silver, platinum, copper, bronze or metal oxides, for instance titanium dioxide, iron oxide or chromium oxide, and mixtures thereof.

The nacreous pigments may be chosen from white nacreous pigments such as mica coated with titanium or with bismuth oxychloride, coloured nacreous pigments such as titanium mica coated with iron oxides, titanium mica coated especially with ferric blue or with chromium oxide, titanium mica coated with an organic pigment of the abovementioned type and also nacreous pigments based on bismuth oxychloride. It is also possible to use interference pigments, especially containing liquid crystals or multilayers.

The liposoluble dyes are, for example, Sudan Red, D & C Red 17, D & C Green 6, β-carotene, soybean oil, Sudan Brown, D & C Yellow 11, D & C Violet 2, D & C Orange 5, quinoline yellow or annatto. The water-soluble dyes are, for example, beetroot juice, methylene blue, the disodium salt of ponceau, the disodium salt of alizarin green, quinoline yellow, the trisodium salt of amaranth, the disodium salt of tartrazine, the monosodium salt of rhodamine, the disodium salt of fuchsin, or xanthophyll.

The composition according to the invention may contain solid particles, such as solid pigments, nacres or fillers, and mixtures thereof, dispersed in the liquid fatty phase and introduced into the composition in the form of a colloidal dispersion, also known as a "particulate paste".

For the purposes of the invention, the expressions "colloidal dispersion" and "particulate paste" mean a concentrated colloidal dispersion of coated or uncoated particles in a continuous medium, surface-stabilized with the aid of a dispersant. These particles may be of any shape, especially of spherical or elongated shape, for instance fibres. They are insoluble in the medium.

The dispersant serves to protect the dispersed particles against their aggregation or flocculation. The dispersant concentration generally used to stabilize a colloidal dispersion is from 0.3 to 5 mg/m² and preferably from 0.5 to 4 mg/m² of surface area of particles. This dispersant may be a surfactant, an oligomer, a polymer or a mixture of several of them, bearing one or more functionalities having a strong affinity for the surface of the particles to be dispersed. In particular, they may attach physically or chemically to the surface of the pigments. These dispersants also contain at least one functional group that is compatible with or soluble in the continuous medium. In particular, esters of 12-hydroxystearic acid in particular and of a C₈ to C₂₀ fatty acid and of a polyol, for instance glycerol or diglycerol, are used, such as the stearate of poly(12-hydroxystearic acid) with a molecular weight of about 750 g/mol, such as the product sold under the name Solsperse 21 000 by the company Avecia, the polyglyceryl-2 dipolyhydroxy-stearate (CTFA name) sold under the reference Dehymyls PGPH by the company Henkel or else polyhydroxystearic acid such as the product sold under the reference Arlacel P100 by the company Uniqema, and mixtures thereof.

As other dispersants which may be used in the composition of the invention, mention may be made of quaternary ammonium derivatives of polycondensed fatty acids, for instance Solsperse 17 000 sold by the company Avecia, and mixtures of polydimethylsiloxane/oxypropylene, such as those sold by the company Dow Corning under the references DC2-5185 and DC2-5225 C.

Polydihydroxystearic acid and the 12-hydroxystearic acid esters are preferably intended for a hydrocarbon-based or fluorinated medium, whereas the mixtures of oxyethylenated/oxypropylenated dimethylsiloxane are preferably intended for a silicone medium.

The colloidal dispersion is a suspension of particles that are generally micron-sized (< 10 µm) in a continuous medium. The volume fraction of particles in a concentrated dispersion is from 20% to 40% and preferably greater than 30%, which corresponds to a weight content that may be up to 70% depending on the density of the particles.

The particles dispersed in the medium may consist of mineral or organic particles or mixtures thereof.

Advantageously, the liquid medium of the particulate paste is one of the oils that it is desired to use in the composition, forming part of the liquid fatty phase.

Advantageously, the "particulate paste" or "colloidal dispersion" is a "pigmentary paste" containing a colloidal dispersion of surface-stabilized coated or uncoated coloured particles. These coloured particles are pigments, nacres or a mixture of pigments and/or nacres.

Advantageously, the colloidal dispersion represents from 0.5% to 60% by weight, better still from 2% to 40% and even better still from 2% to 30% by weight of the composition.

The composition according to the invention may also comprise one or more fillers, especially in a content ranging from 0.01% to 50% by weight and preferably ranging from 0.01% to 30% by weight relative to the total weight of the composition. The term "fillers" should be understood as meaning colourless or white, mineral or synthetic particles of any form, which are insoluble in the medium of the composition irrespective of the temperature at which the composition is manufactured. These fillers serve especially to modify the rheology or texture of the composition.

The fillers may be mineral or organic of any shape, lamellar, spherical or oblong. Mention may be made of talc, mica, silica, kaolin, polyamide powder for instance Nylon® (Orgasol from Atochem), poly-β-alanine powder and polyethylene powder, tetrafluoroethylene polymer powders for instance Teflon® , lauroyllysine, starch, boron nitride, expanded hollow polymer microspheres such as those made of polyvinylidene chloride/acrylonitrile, for instance Expancel® (Nobel Industrie), acrylic powders such as Polytrap® (Dow Corning), polymethyl methacrylate particles and silicone resin microbeads (for example Tospearls® from Toshiba), precipitated calcium carbonate, magnesium carbonate, magnesium hydrocarbonate, hydroxyapatite, hollow silica microspheres (Silica Beads® from Maprecos), glass or ceramic microcapsules, and metal soaps derived from organic carboxylic acids containing from 8 to 22 carbon atoms and preferably from 12 to 18 carbon atoms, for example zinc, magnesium or lithium stearate, zinc laurate or magnesium myristate and mixtures thereof.

The fillers may represent from 0.1% to 25% and better still from 1% to 20% by weight relative to the total weight of the composition.

The composition according to the invention may also contain ingredients commonly used in cosmetics, such as vitamins, trace elements, softeners, sequestering agents, fragrances, acidifying or basifying agents, preserving agents, sunscreens, antioxidants, fibres, agents for preventing hair loss, eyelash care agents, antidandruff agents and propellants, or mixtures thereof.

The term "fibre" should be understood as meaning an object of length L and diameter D such that L is very much greater than D, D being the diameter of the circle within which the cross section of the fibre is inscribed. In particular, the ratio L/D (or form factor) is chosen within the range from 3.5 to 2500, preferably from 5 to 500 and better still from 5 to 150.

In particular, the fibres have a length ranging from 1 µm to 10 mm, preferably from 0.1 mm to 5 mm and better still from 0.3 mm to 3 mm.

The fibres that may be used in the composition of the invention may be chosen from rigid and non-rigid fibres, and they may be of synthetic or natural, mineral or organic origin.

As fibres that may be used in the composition according to the invention, mention may be made of non-rigid fibres such as polyamide (Nylon® ) fibres, polyethylene fibres or cellulose (rayon) fibres, such as those sold by the company Claremont Flock "Natural rayon flock fibre RC1BE - N003 - M04", or rigid fibres, such as polyimide-amide fibres, for instance those sold under the name "Kermel" or "Kermel Tech" by the company Rhodia, or poly(p-phenyleneterephthalamide) (or aramid) fibres, sold especially under the name Kevlar® by the company DuPont de Nemours.

The fibres may be present in the composition according to the invention in a content ranging from 0.1% to 10% by weight and better still from 0.5% to 5% by weight relative to the total weight of the composition.

According to one embodiment, the composition according to the invention is free of antimicrobial agents.

Needless to say, a person skilled in the art will take care to select this or these optional additional compound(s), and/or the amount thereof, such that the advantageous properties of the corresponding composition according to the invention are not, or are not substantially, adversely affected by the envisaged addition.

The composition according to the invention may especially be in the form of a stick, a suspension, a dispersion, a solution, a gel, an emulsion, especially an oil-in-water (O/W) or water-in-oil (W/O) emulsion, or a multiple emulsion (W/O/W or polyol/O/W or O/W/O), in the form of a cream, a paste, a mousse, a dispersion of vesicles, especially of ionic or nonionic lipids, a two-phase or multi-phase lotion, a spray, a powder, a paste (especially a soft paste (especially a paste with a dynamic viscosity at 25°C of about from 0.1 to 40 Pa.s under a shear rate of 200 s⁻¹, after 10 minutes of measurement in cone/plate geometry). The composition may be anhydrous; for example, it may be an anhydrous paste.

A person skilled in the art may select the appropriate galenical form, and also the method for preparing it, on the basis of his general knowledge, taking into account firstly the nature of the constituents used, especially their solubility in the support, and secondly the intended use of the composition.

The composition according to the invention may be a makeup composition, for instance products for complexion (foundations), makeup rouges, eyeshadows, lipsticks, concealer products, blushers, mascaras, eyeliners, eyebrow makeup products, lip pencils, eye pencils, nail products, such as nail varnishes, body makeup products or hair makeup products (hair mascara or hair lacquer).

The composition according to the invention may also be a skincare product for body and facial skin, especially an antisun product or a skin colouring product (such as a self-tanning product).

A subject of the present invention is also a cosmetic assembly comprising:
- a container delimiting at least one compartment, the said container being closed by means of a closing member; and
- a composition as described above, placed inside the said compartment.

The container may be in any adequate form. It may especially be in the form of a bottle, a tube, a jar, a case, a box, a sachet or a carton.

The closing member may be in the form of a removable stopper, a lid, a cap, a tear-off strip or a capsule, especially of the type comprising a body attached to the container and a cover cap articulated on the body. It may also be in the form of a member for selectively closing the container, especially a pump, a valve or a flap valve.

The container may be combined with an applicator, especially in the form of a brush comprising an arrangement of bristles maintained by a twisted wire. Such a twisted brush is described especially in patent US 4 887 622. It may also be in the form of a comb comprising a plurality of application members, obtained especially by moulding. Such combs are described, for example, in patent FR 2 796 529. The applicator may be in the form of a fine brush, as described, for example, in patent FR 2 722 380. The applicator may be in the form of a block of foam or of elastomer, a felt or a spatula. The applicator may be free (tuft or sponge) or securely fastened to a rod borne by the closing member, as described, for example, in patent US 5 492 426. The applicator may be securely fastened to the container, as described, for example, in patent FR 2 761 959.

The product may be contained directly in the container, or indirectly. By way of example, the product may be arranged on an impregnated support, especially in the form of a wipe or a pad, and arranged (individually or in plurality) in a box or in a sachet. Such a support incorporating the product is described, for example, in patent application WO 01/03538.

The closing member may be coupled to the container by screwing. Alternatively, the coupling between the closing member and the container is done other than by screwing, especially via a bayonet mechanism, by click-fastening, gripping, welding, bonding or by magnetic attraction. The term "click-fastening" in particular means any system involving the crossing of a bead or cord of material by elastic deformation of a portion, especially of the closing member, followed by return to the elastically unconstrained position of the said portion after the crossing of the bead or cord.

The container may be at least partially made of thermoplastic material. Examples of thermoplastic materials that may be mentioned include polypropylene or polyethylene.

Alternatively, the container is made of non-thermoplastic material, especially glass or metal (or alloy).

The container may have rigid walls or deformable walls, especially in the form of a tube or a tubular bottle.

The container may comprise means for distributing or facilitating the distribution of the composition. By way of example, the container may have deformable walls so as to allow the composition to exit in response to a positive pressure inside the container, this positive pressure being caused by elastic (or non-elastic) squeezing of the walls of the container. Alternatively, especially when the product is in the form of a stick, the product may be driven out by a piston mechanism. Still in the case of a stick, especially of makeup product (lipstick, foundation, etc.), the container may comprise a mechanism, especially a rack mechanism, a threaded-rod mechanism or a helical groove mechanism, and may be capable of moving a stick in the direction of the said aperture. Such a mechanism is described, for example, in patent FR 2 806 273 or in patent FR 2 775 566. Such a mechanism for a liquid product is described in patent FR 2 727 609.

The container may consist of a carton with a base delimiting at least one housing containing the composition, and a lid, especially articulated on the base, and capable of at least partially covering the said base. Such a carton is described, for example, in patent application WO 03/018423 or in patent FR 2 791 042.

The container may be equipped with a drainer arranged in the region of the aperture of the container. Such a drainer makes it possible to wipe the applicator and possibly the rod to which it may be securely fastened. Such a drainer is described, for example, in patent FR 2 792 618.

The composition may be at atmospheric pressure inside the container (at room temperature) or pressurized, especially by means of a propellent gas (aerosol). In the latter case, the container is equipped with a valve (of the type used for aerosols).

The content of the patents or patent applications mentioned above are incorporated by reference into the present patent application.

The examples that follow illustrate the compositions according to the invention in a nonlimiting manner.

Unless otherwise mentioned, the amounts indicated are expressed in grams.

### Example 1: Mascara

One embodiment of the invention may be the following mascara composition:
- 2-Amino-2-methylpropane-1,3-diol 0.5
- Stearic acid 5.82
- Carnauba wax 2.9
- Beeswax 3.7
- Polytetrafluoroethylene wax 2
- Paraffin wax 11.8
- D-Panthenol 0.45
- Black iron oxide 7.2
- Hydroxyethylcellulose quaternized with 2,3- 0.08 epoxypropyltrimethylammonium chloride
- Polyethylene glycol stearate (40 OE) 0.5 (Myrj 52 P from Uniqema)
- Hydroxyethylcellulose 0.93
- Mixture of polydimethylsiloxane and of hydrated 0.13 silica
- Non-stabilized sodium polymethacrylate at 25% 0.9 in water (Darvan 7 from Vanderbilt)
- Gum arabic 3.46
- Hydroxypropyl chitosan 0.12
- Triethanolamine 2.4
- Cellulose fibre ("Natural rayon flock fibre 0.5 RC1BE - N003 - M04" from Claremont Flock)
- Talc 2
- Acrylic copolymer (ACP 10 from 3M) in phenyl 0.2 trimethicone (sold under the reference MSX 5381 by 3M)
- Preserving agents qs
- Water qs 100

### Example 2: Lipstick

One embodiment of the invention may be the following lipstick:

| | |
|---|---|
| Polyglyceryl isostearate (Salacos 41 from Nisshin Oils Mills) | 10 |
| Polyethylene wax | 2 |
| Diisostearyl malate | 16.1 |
| Glyceryl triacetyl hydroxystearate | 10 |
| Dipentaerythrityl tetrahydroxystearate | 10 |
| BHT | 0.05 |
| Hydrogenated cocoglycerides | 5 |
| Microcrystalline wax SP 18 from Strahl & Pitsch | 1 |
| Acrylic copolymer (ACP 10 from 3M) in phenyl trimethicone (sold under the reference MSX 5381 by 3M) | 5.75 |
| Phenyl trimethicone (DC 556 from Dow Corning) | 5.7 |
| Pure jojoba oil (from Desert Whale) | 0.2 |
| Pigments | 6 |
| Isopropyl isononanoate | qs 100 |

## Claims

1. Cosmetic composition comprising, in a cosmetically acceptable medium:
- a polymer resulting from the copolymerization:
a) of at least one monomer A chosen from esters derived from the reaction of (meth)acrylic acid with a monoalcohol containing from 2 to 20 carbon atoms,
b) of at least one monomer B chosen from esters derived from the reaction of methacrylic acid with a monoalcohol containing from 1 to 10 carbon atoms, and
c) of at least one monomer C chosen from
N-vinyllactams and derivatives thereof, and
- an organic solvent phase comprising at least one first organic solvent, the said phase being free of lower monoalcohol containing from 1 to 5 carbon atoms or of C₃-C₄ ketone, or containing them in a weight content, relative to the total weight of the composition, of less than or equal to 15%.

2. Cosmetic composition comprising, in a cosmetically acceptable medium:
- a polymer resulting from the copolymerization:
d) of at least one monomer A chosen from esters derived from the reaction of (meth)acrylic acid with a monoalcohol containing from 2 to 20 carbon atoms,
e) of at least one monomer B chosen from esters derived from the reaction of methacrylic acid with a monoalcohol containing from 1 to 10 carbon atoms, and
f) of at least one monomer C chosen from
N-vinyllactams and derivatives thereof, and
- at least one additional film-forming polymer.

3. Composition according to Claim 1 or 2, **characterized in that** the polymer comprises at least one monomer A resulting from the reaction of methacrylic acid with a monoalcohol containing from 5 to 20 carbon atoms, preferably from 7 to 18 carbon atoms and better still from 10 to 18 carbon atoms.

4. Composition according to Claim 3, **characterized in that** the monoalcohol is chosen from: 3-heptanol, 1-octanol, 2-octanol, isooctyl alcohol, 2-ethyl-1-hexanol, 1-decanol, 1-dodecanol, 1-tridecanol, 1-tetradecanol and 1-octadecanol, and mixtures thereof.

5. Composition according to one of the preceding claims, **characterized in that** the polymer comprises at least one monomer A resulting from the reaction of acrylic acid with a monoalcohol containing from 2 to 15 carbon atoms and preferably from 4 to 14 carbon atoms.

6. Composition according to Claim 5, **characterized in that** the C2-C15 monoalcohol is chosen from: ethanol, 1-butanol, 2-butanol, 1-pentanol, 2-pentanol, 3-pentanol, 2-methyl-1-butanol, 1-hexanol, 2-hexanol, 2-methyl-1-pentanol, 3-methyl-1-pentanol, 2-ethyl-1-butanol, 3,5,5-trimethyl-1-hexanol, 3-heptanol, 1-octanol, 2-octanol, isooctyl alcohol, 2-ethyl-1-hexanol, 1-decanol, 1-dodecanol, 1-tridecanol and 1-tetradecanol, and mixtures thereof.

7. Composition according to one of the preceding claims, **characterized in that** the monomer A is chosen from n-butyl acrylate, isooctyl acrylate and lauryl methacrylate, and mixtures thereof.

8. Composition according to one of the preceding claims, **characterized in that** the monomer A is present in a numerical proportion ranging from 15% to 80% and better still from 40% to 60% relative to the total number of monomers in the polymer.

9. Composition according to one of the preceding claims, **characterized in that** the monomer B is chosen from esters derived from the reaction of methacrylic acid with a monoalcohol containing from 1 to 6 and better still from 1 to 4 carbon atoms.

10. Composition according to one of the preceding claims, **characterized in that** the monoalcohol is chosen from methanol, ethanol, 1-propanol, 2-propanol, 1-butanol, 2-butanol, 1-pentanol, 2-pentanol and 3-pentanol, and mixtures thereof.

11. Composition according to one of the preceding claims, **characterized in that** the monomer B is chosen from methyl methacrylate and n-butyl methacrylate, and mixtures thereof.

12. Composition according to one of the preceding claims, **characterized in that** the monomer B is present in a numerical proportion ranging from 20% to 70% and better still from 25% to 50% relative to the total number of monomers in the polymer.

13. Composition according to one of the preceding claims, **characterized in that** the monomer C is chosen from the N-vinyllactams having the following formula: 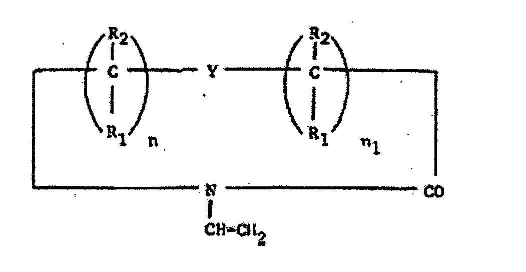 in which:
R1 and R2 independently represent a hydrogen atom, a C₁₋C₅ alkyl group such as methyl, ethyl or propyl, or an aryl group,
Y is chosen from O, S, -SO₂-, 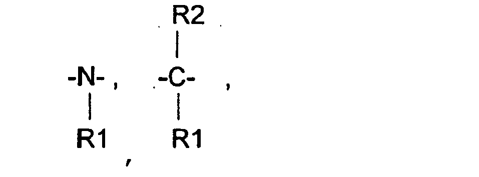 and n and n1 range from 0 to 5, with the proviso that n and n1 are not simultaneously equal to 0.

14. Composition according to one of the preceding claims, **characterized in that** the monomer C is chosen from N-vinylpyrrolidone and the N-vinyl-substituted derivatives of the following lactams: 3,3-dimethyl-1-pyrrolidone, 4,4-dimethyl-2-pyrrolidone, 3,4-dimethyl-2-pyrrolidone, 3-ethyl-2-pyrrolidone and 3,5-dimethyl-2-pyrrolidone.

15. Composition according to one of the preceding claims, **characterized in that** the monomer C is present in a numerical proportion ranging from 1% to 15% and better still from 5% to 15% relative to the total number of monomers in the polymer.

16. Composition according to one of the preceding claims, **characterized in that** the polymer represents from 0.01% to 30% by weight, preferably from 0.05% to 20% by weight and better still from 0.05% to 15% by weight of solids (or active material), relative to the total weight of the composition.

17. Composition according to Claim 2, **characterized in that** it comprises an organic solvent phase comprising at least one first organic solvent.

18. Composition according to one of the preceding claims, **characterized in that** the first organic solvent is chosen from volatile or non-volatile hydrocarbon-based oils and silicone oils, and mixtures thereof.

19. Composition according to one of the preceding claims, **characterized in that** the organic solvent phase represents from 0.05% to 98%, preferably from 0.1% to 70% and better still from 0.5% to 60% by weight relative to the total weight of the composition.

20. Composition according to one of the preceding claims, **characterized in that** the organic solvent phase comprises a lower monoalcohol containing from 1 to 5 carbon atoms or a C₃-C₉ ketone in a weight content of less than or equal to 10% and preferably less than or equal to 5% by weight relative to the total weight of the composition.

21. Composition according to one of Claims 1 to 19, **characterized in that** it is free of lower monoalcohols containing from 1 to 5 carbon atoms or of C₃-C₄ ketone.

22. Composition according to one of the preceding claims, **characterized in that** it comprises an aqueous phase.

23. Composition according to the preceding claim, **characterized in that** the aqueous phase is present in the composition according to the invention in a content ranging from 1% to 95% by weight and preferably ranging from 1% to 80% by weight relative to the total weight of the composition.

24. Composition according to either of Claims 22 and 23, **characterized in that** it comprises a hydrophilic or water-soluble gelling and/or film-forming polymer.

25. Composition according to the preceding claim, **characterized in that** the gelling agent is present in a content ranging from 0.05% to 40% by weight, preferably from 0.5% to 20% and better still from 1% to 15% by weight relative to the total weight of the composition.

26. Composition according to one of the preceding claims, **characterized in that** it comprises a surfactant.

27. Composition according to the preceding claim, **characterized in that** the surfactant is present in a proportion ranging from 0.5% to 30%, better still from 2% to 20% and even better still from 3% to 15% by weight relative to the total weight of the composition.

28. Composition according to one of the preceding claims, **characterized in that** it comprises at least one additional film-forming polymer.

29. Composition according to the preceding claim, **characterized in that** the additional film-forming polymer is present in a solids content ranging from 0.1% to 60% by weight, preferably from 0.5% to 40% by weight and better still from 1% to 30% by weight relative to the total weight of the composition.

30. Composition according to any one of the preceding claims, **characterized in that** it comprises at least one fatty substance that is solid at room temperature, chosen from waxes, pasty fatty substances and gums, and mixtures thereof.

31. Composition according to the preceding claim, **characterized in that** the wax is chosen from beeswax, lanolin wax, Chinese insect waxes, rice wax, carnauba wax, candelilla wax, ouricury wax, esparto grass wax, cork fibre wax, sugarcane wax, Japan wax and sumach wax; montan wax, microcrystalline waxes, paraffins and ozokerite; polyethylene waxes, the waxes obtained by Fisher-Tropsch synthesis and waxy copolymers, and also esters thereof, the waxes obtained by catalytic hydrogenation of animal or plant oils containing linear or branched C8-C32 fatty chains, the wax obtained by hydrogenation of olive oil esterified with stearyl alcohol, silicone waxes, fluoro waxes, waxes with a tack of greater than or equal to 0.7 N.s and a hardness of less than or equal to 3.5 MPa, and mixtures thereof.

32. Composition according to Claim 30 or 31, **characterized in that** it contains from 0.1% to 50% by weight, better still from 1% to 40% and even better still from 5% to 30% by weight of waxes, relative to the total weight of the composition.

33. Cosmetic composition according to any one of the preceding claims, **characterized in that** it comprises one or more dyestuffs chosen especially from water-soluble dyes and pulverulent dyestuffs, such as pigments, nacres and flakes or other particles with an optical effect.

34. Composition according to any one of the preceding claims, **characterized in that** it comprises a filler.

35. Composition according to the preceding claim, **characterized in that** the filler is chosen from talc, mica, silica, kaolin, polyamide powder, poly-β-alanine powder and polyethylene powder, tetrafluoroethylene polymer powders, lauroyllysine, starch, boron nitride, expanded hollow polymer microspheres, acrylic acid copolymers and silicone resin microbeads, elastomeric polyorganosiloxane particles, precipitated calcium carbonate, magnesium carbonate, magnesium hydrocarbonate, hydroxyapatite, hollow silica microspheres, glass or ceramic microcapsules, and metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate, and mixtures thereof.

36. Composition according to one of the preceding claims, **characterized in that** the filler represents from 0.1% to 25% and better still from 1% to 20% by weight relative to the total weight of the composition.

37. Composition according to any one of the preceding claims, **characterized in that** it comprises a cosmetic ingredient chosen from vitamins, trace elements, softeners, sequestering agents, fragrances, acidifying agents or basifying agents, preserving agents, sunscreens, antioxidants, fibres, agents for preventing hair loss, eyelash care agents, antidandruff agents and propellants, or mixtures thereof.

38. Cosmetic composition according to any one of the preceding claims, **characterized in that** it is in the form of a suspension, a dispersion, a solution, a gel, an emulsion, especially an oil-in-water (O/W) or water-in-oil (W/O) emulsion, or a multiple emulsion (W/O/W or polyol/O/W or O/W/O), in the form of a cream, a stick, a paste, a mousse, a dispersion of vesicles, especially of ionic or nonionic lipids, a two-phase or multi-phase lotion, a spray, a powder or a paste, especially a soft paste or an anhydrous paste.

39. Cosmetic composition according to any one of the preceding claims, **characterized in that** it is a makeup or care composition for keratin materials.

40. Composition according to one of Claims 1 to 39, **characterized in that** it is a makeup product for the eyes.

41. Composition according to one of Claims 1 to 40, **characterized in that** it is a mascara.

42. Composition according to one of Claims 1 to 41, **characterized in that** it is a makeup product for the lips.

43. Cosmetic assembly comprising:
a) a container delimiting at least one compartment, the said container being closed by means of a closing member; and
b) a composition placed inside the said compartment, the composition being in accordance with any one of Claims 1 to 42.

44. Cosmetic assembly according to Claim 43, **characterized in that** the container is at least partially formed from at least one thermoplastic material.

45. Cosmetic assembly according to Claim 43, **characterized in that** the container is at least partially formed from at least one non-thermoplastic material, especially from glass or metal.

46. Assembly according to any one of Claims 43 to 45, **characterized in that**, in the closed position of the container, the closing member is screwed onto the container.

47. Assembly according to any one of Claims 43 to 45, **characterized in that**, in the closed position of the container, the closing member is coupled to the container other than by screwing, especially by click-fastening, bonding or welding.

48. Assembly according to any one of Claims 43 to 47, **characterized in that** the composition is substantially at atmospheric pressure inside the compartment.

49. Assembly according to any one of Claims 43 to 47, **characterized in that** the composition is pressurized inside the container.

50. Cosmetic process for making up or caring for keratin materials, comprising the application to the keratin materials of a cosmetic composition according to one of Claims 1 to 42.

51. Use of a polymer resulting from the copolymerization:
a) of at least one monomer A chosen from esters derived from the reaction of (meth)acrylic acid with a monoalcohol containing from 2 to 20 carbon atoms,
b) of at least one monomer B chosen from esters derived from the reaction of methacrylic acid with a monoalcohol containing from 1 to 10 carbon atoms, and
c) of at least one monomer C chosen from N-vinyllactams
to prepare a cosmetic composition capable of forming on keratin materials a film that has lengthening properties and/or good staying power and/or migration-resistance and/or transfer-resistance.
